# EUROPÄISCHE PATENTSCHRIFT

(11) **EP 1 103 483 B1**
(45) Veröffentlichungstag und Bekanntmachung des Hinweises auf die Patenterteilung: **22.12.2004**
(21) Anmeldenummer: 00116371.6
(22) Anmeldetag: 28.07.2000
(51) Int. Cl.: B65D 75/30, A61L 9/12, B29C 65/52, B29C 65/00, D06H 5/00

(54) **Verfahren zur Herstellung einer Beutelverpackung für duftende Produkte sowie Beutelverpackung**
Method for making bag-like packages for perfumed products and bag-like package
Procédé de fabrication d'une pochette contenant un produit parfumé et pochette

(30) Priorität: 29.10.1999 DE 19952499
(43) Veröffentlichungstag der Anmeldung: 30.05.2001
(73) Patentinhaber: August Töpfer & Co. (GmbH & Co.), 20539 Hamburg (DE); Henkel KGaA, 40589 Düsseldorf (DE)
(72) Erfinder: Dipl.-Ing, Rudolf Ernst, D-22607 Hamburg (DE); Gräf, Bernd, 40699 Erkrath (DE)
(74) Vertreter: von Raffay, Vincenz, Dipl.-Ing.

(56) Entgegenhaltungen:
- FR-A- 2 449 743
- FR-A- 2 702 961
- US-A- 2 106 875
- US-A- 4 880 690
- US-A- 5 613 601

## Beschreibung

Die vorliegende Erfindung betrifft ein Verfahren zur Herstellung einer Beutelverpackung für duftende Stoffe, sowie eine durch dieses Verfahren hergestellte Beutelverpackung.

Die FR-A-2 72 961 zeigt einen Duftbeutel aus nicht-gewobenem Material, das im Bereich der Nähte mit Siegellack bedruckt wird (Seite 5 Zeilen 26-32).

Die US-A-2 106 875 zeigt, wie zwei Gewebeschichten mit Siegellack, der ins Gewebe einsickert, miteinander verbunden werden.

Bei den hier angesprochenen Beuteln handelt es sich um sogenannte Duftbeutel, die aus einem porösen Gewebe bestehen, damit die Duftstoffe aus dem eingefüllten Produkt, einem Duftgranulat oder dgl. in die Umgebung entweichen können. Diese Duftbeutel werden aus Gründen der Lagerfähigkeit in einem gasdichten Umbeutel weiter verpackt. Der Verbraucher öffnet den Umbeutel und hängt den entnommenen Duftbeutel, beispielsweise in einen Wäscheschrank. Der Duftbeutel weist einen Schlitz auf, damit man einen Faden zum Aufhängen durchziehen kann.

Es gibt nun viele Materialien, insbesondere Viskose, die nicht schweiß- oder siegelfähig sind, um die Verbindungsnähte zur Bildung des Beutels herzustellen. Der Erfindung liegt daher die Aufgabe zugrunde, ein Verfahren und eine Verpackung der eingangs genannten Art zu schaffen, die auch aus Gewebematerialien, insbesondere Viskose, hergestellt werden können, die sich nicht durch Schweißen oder dgl. zu einem Beutel miteinander verbinden lassen. Anders ausgedrückt, es soll ein Verfahren der eingangs genannten Art geschaffen werden, durch das eine Beutelverpackung auf konventionellen Verpackungsmaschinen hergestellt werden kann.

Diese Aufgabe wird bei einem Verfahren nach dem Kennzeichen des Anspruches 1 und bei dem entsprechenden Beutel nach dem Kennzeichen des Anspruches 2 gelöst.

Insbesondere hat es sich als erfindungsgemäß wichtig herausgestellt, den Siegellack in mehreren Schichten aufzubringen und zwischen diesem Schichtauftrag zu trocknen.

Erfindungsgemäß wird das textile Gewebe, d.h. insbesondere die Viskose, mit einem Siegellack beschichtet und zwar nur dort, wo die Verbindung erfolgen soll. Die übrigen Bereiche, in denen die Duftstoffe austreten sollen, bleiben unbehandelt. Die Beschichtung erfolgt auf einer Mehrfachdruckmaschine. Die Trocknung verhindert, daß der Siegellack zu stark in das Gewebe eindringt und auf diese Weise verloren geht.

Grundsätzlich ist es also so, daß durch die Erfindung eine Herstellung aus textilem Gewebe auf Siegelmaschinen möglich ist, wobei der Siegelauftrag mehrschichtig mit einer Zwischentrocknung erfolgt, wobei der Siegellack nur im Bereich der Nähte vorgesehen ist.

Beim Siegeln wird im Unterschied zum Schweißen die aufgebrachte Siegelschicht verschweißt und nicht das Material, in diesem Falle das textile Gewebe, das mit dem Siegellack beschichtet wurde. Beim Schweißen wird das Material selbst verflüssigt.

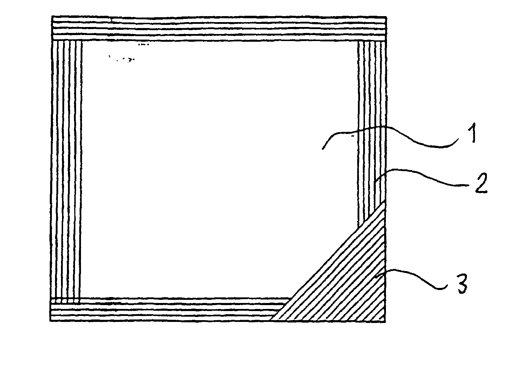

In der einzigen Figur ist eine Ausführungsform einer Beutelverpackung in Draufsicht schematisch dargestellt. Das textile Gewebe 1 wird im Bereich der Nähte mit Siegellack 2 bedruckt. Eine verstärkte Ecke ist mit 3 bezeichnet. Das auf diese Weise behandelte Gewebe kann auf einer herkömmlichen Siegelmaschine verarbeitet und zu dem Beutel zusammengefügt werden.

Als Siegellack hat sich ein Dispersionslack, der ein Acryllack ist, bewährt. Dieser Lack enthält Bindemittel, Wachse, Additive und Wasser. Der Siedepunkt liegt bei ca. 100°C.

## Patentansprüche

1. Verfahren zur Herstellung einer Beutelverpackung für duftende Produkte, **dadurch gekennzeichnet, daß** ein textiles Gewebe, insbesondere Viskose, im Bereich der Nähte zur Bildung der Beutelform mit Siegellack bedruckt und anschließend die zur Verbindung vorgesehenen Siegelnähte miteinander versiegelt werden und
dass der Siegellack in mehreren Schichten aufgebracht wird, und
dass zwischen dem Aufbringen der einzelnen Siegelnähte eine Trocknung erfolgt.

2. Verpackung für duftende Produkte, **gekennzeichnet durch** die Herstellung aus einem textilen Gewebe (1), insbesondere Viskose, das im Bereich der Nähte (2) zur Bildung der Beutelform mit Siegellack beschichtet ist, und
dass der Siegellack im Bereich der Nähte in mehreren Schichten aufgetragen ist, und
dass die Siegellackschichten zwischen den einzelnen Auftragsvorgängen getrocknet sind.

## Claims

1. Method for the manufacture of a pouch for fragrant products, **characterized in that** a textile fabric, particularly viscose, is impressed with sealing wax in the vicinity of the seams for forming the bag shape and subsequently the sealing seams provided for joining are sealed together and that the sealing wax is applied in a number of coatings and that drying takes place between the application of the individual sealing seams.

2. Pouch for fragrant products, **characterized by** manufacture from a textile fabric (1), particularly viscose, which is coated with sealing wax in the vicinity of the seams (2) for forming the bag shape and that, in the vicinity of the seams, the sealing wax is applied in a number of coatings and that the sealing wax coatings are dried between the individual application processes.

## Revendications

1. Procédé destiné à la fabrication d'un conditionnement en sachet pour produits parfumés, **se caractérisant par le fait qu'**un tissu textile, en particulier de la viscose, sera imprimé d'une laque de scellage dans la zone de la ligne de joint afin d'obtenir la forme en sachet et ensuite que les lignes de joint scellées prévues pour la jonction sont scellées les unes aux autres et que la laque de scellage est appliquée sur plusieurs couches et qu'un séchage a lieu entre l'application des différentes lignes de joint.

2. Emballage de produits parfumés **se caractérisant par** la fabrication à partir d'un tissu textile (1), en particulier de la viscose qui est revêtue de laque de scellage dans la zone des lignes de joint (2) afin d'obtenir la forme d'un sachet, et
que la laque de scellage est appliquée dans la zone des lignes de joint en plusieurs couches et
que les couches de laque de scellage sont séchées entre les différentes applications.
